# EUROPEAN PATENT SPECIFICATION

(11) **EP 2 261 259 B1**
(45) Date of publication and mention of the grant of the patent: **30.07.2014**
(21) Application number: 09162439.5
(22) Date of filing: 10.06.2009
(51) Int. Cl.: G01N 33/94, C07K 16/44

(54) **Immunoassay for N-(3-chlorophenyl)piperazine-based anti-depressants**
Immunoassay für N-(3-chlorphenyl)piperazin-basierte Antidepressiva
Dosage immunologique pour anti-dépresseurs base de N-(3-chlorophényl)pipérazine

(43) Date of publication of application: 15.12.2010
(73) Proprietor: Randox Laboratories Ltd., Crumlin, County Antrim BT29 4QY (GB)
(72) Inventor: Fitzgerald, Stephen, Crumlin, Antrim BT29 4QY (GB); Benchikh, Elouard, Crumlin, Antrim BT29 4QY (GB); McConnell, Ivan, Crumlin, Antrim BT29 4QY (GB); Lowry, Philip, Crumlin, Antrim BT29 4QY (GB)

(56) References cited:
- WO-A-97/19950
- DE BOER DOUWE ET AL: "Piperazine-like compounds: A new group of designer drugs-of-abuse on the European market" FORENSIC SCIENCE INTERNATIONAL, vol. 121, no. 1-2, September 2001 (2001-09), pages 47-56, XP002538332 ISSN: 0379-0738
- SEGUELA P ET AL: "ANTIBODIES AGAINST GAMMA AMINO BUTYRIC-ACID SPECIFICITY STUDIES AND IMMUNO CYTOCHEMICAL RESULTS" PROCEEDINGS OF THE NATIONAL ACADEMY OF SCIENCES OF THE UNITED STATES OF AMERICA, vol. 81, no. 12, 1984, pages 3888-3892, XP002538333 ISSN: 0027-8424
- KING L.A.: "m-chlorophenylpiperazine (mCPP)" [Online] 2007, EMCDDA , LISBON , XP002538334 Retrieved from the Internet: URL:http://www.emcdda.europa.eu/attachemen ts.cfm/att_33257_EN_Annex_1_mCPP_Technical _Information.pdf> [retrieved on 2009-07-21] * the whole document *

## Description

### BACKGROUND TO THE INVENTION

Trazodone, systematic name 2-{3-[4-(3-chlorophenyl)-1-piperazinyl]propyl}-[1,2,4]triazolo[4,3-a]pyridin-3(2*H*)-one, is an anti-depressant drug with anxiolytic and hypnotic activity which is metabolized in the liver by hydroxylation, N-oxidation and dealkylation, the latter transformation giving rise to a major metabolite N-(3-chlorophenyl)piperazine. Acute intake of trazodone, usually when taken with other drugs, has been associated with toxicity and fatal overdose (e.g. Balestrieri et al 1992; Goeringer et al 2000; Haria et al. 1994; de Meester et al 2001; Martinez et al 2005).

Nefazodone, systematic name 2-{3-[4-(3-chlorophenyl)-1-piperazinyl]propyl}-5-ethyl -4-(2-phenoxyethyl)- 1,2,4-triazol-3-one, is an anti-depressant drug that has been associated with liver-related adverse reactions and fatalities, and serotonin syndrome (Mayol et al 1994; John et al 1997). N-(3-chlorophenyl)piperazine is detected in urine within 24 hours of nefazodone intake but the major urinary metabolites are nefazodone metabolites devoid of the N-(3-chlorophenyl)piperazine structure. The major plasma metabolite of nefazodone is 1-{3-[4-(3-chlorophenyl)-1-piperazinyl]propyl}-4-(2-phenoxyethyl)- 1,2,4-triazol-3,5-dione (Punit et al 1996). Other N'-substituted- N-(3-chlorophenyl)piperazines include etoperidone, mepiprazole, cloperidone and mefeclorazine.

The potential for adverse and fatal events in individuals taking, or with access to, trazodone and nefazodone, means that there is a need in clinical and forensic toxicology for their detection and/or determination using practical and inexpensive analytical methods. Analytical methods that have been used to detect and determine trazodone, nefazodone and their metabolites include HPLC, HPLC-MS, GC, GC-MS and molecule-selective electrodes. N-(3-chlorophenyl)piperazine as a drug of misuse, including methods of detection, is reviewed in a publication commissioned by the EMCDDA (2007).

Specific binding reactions, such as antibody-antigen interactions, have been used extensively in immunoassays to detect a variety of substances present in biological samples. Compared to methods such as HPLC and GC-MS, such methods are less costly, require non-specialist staff for implementation and can be applied outside of the laboratory e.g. incorporated into a dipstick device. Thus, radioimmunoassays (RIAs) could be used for the determination of trazodone, nefazodone and molecules comprising or containing the N-(3-chlorophenyl)piperazine or N'-substituted- N-(3-chlorophenyl)piperazine structures such as mepiprazole and etoperidone. Radioimmunoassays are very sensitive, but do require radionuclide tracers, for example ¹²⁵I and ³H. Enzyme-linked immunosorbent assays (ELISAs) are a nonradioactive alternative that could be used for their qualitative and quantitative determination. There are no known RIAs or ELISAs for trazodone, nefazodone or any other molecule comprising or containing the N-(3-chlorophenyl)piperazine or N'-substituted- N-(3-chlorophenyl)piperazines structures. De Boer et al (2001) explore the analysis of the structurally diverse (i.e. molecules not containing the N-(3-chlorophenyl)piperazine structure) 1-benzylpiperazine (BZP), 1-(4-methoxyphenyl)piperazine and 1-(3-trifluoromethylphenyl)piperazine using various analytical techniques including commercially available immunoassays for amphetamines. One of the immunoassays showed a very low cross-reactivity to high concentrations of BZP.

To enable drug screening of trazodone, nefazodone and related molecules comprising or containing N-(3-chlorophenyl)piperazine or N'-substituted- N-(3-chlorophenyl)piperazine structures for clinical and forensic toxicology purposes, an economically viable, practical, sensitive and robust test is required. The invention described herein possesses these attributes.

### Bibliography

Balestrieri G. et al (1992). Br. Med. J., 304: 686.
Goeringer K.E. et al (2000). J. Forensic Sci., 45: 850-856.
Mayol R.F. et al (1994). Drug Metab. Dispos., 22: 304-311.
de Meester A. et al (2001). Act. Clin. Belg., 56: 258-261.
Haria M. et al (1994). Drugs and Aging, 4: 331-335.
Punit M.H. (1996). Br. J. Clin. Pharmacol., 41: 21-27.
John L. (1997). Ann. Emerg. Med., 29: 287-289.
Martinez M.A. et al (2005). J. Anal. Toxicol. , 29: 262-268.
de Boer D. et al (2001). Forensic Sci. Int., 121: 47-56.
EMCDDA (2007). Annex 1 Technical Information: m-Chlorophenylpiperazine (mCPP), in Europol-EMCDDA active monitoring report on a new psychoactive substance 1-(3-chlorophenyl)piperazine (mCPP), ed. European Monitoring Centre for Drugs and Drug Addiction.
Seguela P. et al. (1984). Proc. Natl. Acad. Sci., 81: 3888-3892.

### SUMMARY OF THE INVENTION

The invention provides a solution to the problem of a lack of an analytical test for trazodone, nefazodone, N-(3-chlorophenyl)piperazine and N'-substituted- N-(3-chlorophenyl)piperazines that is economically viable, practical, sensitive and robust. The solution requires the use of a unique antibody that binds to N-(3-chlorophenyl)piperazine and N'-substituted- N-(3-chlorophenyl)piperazines.

### DRAWINGS

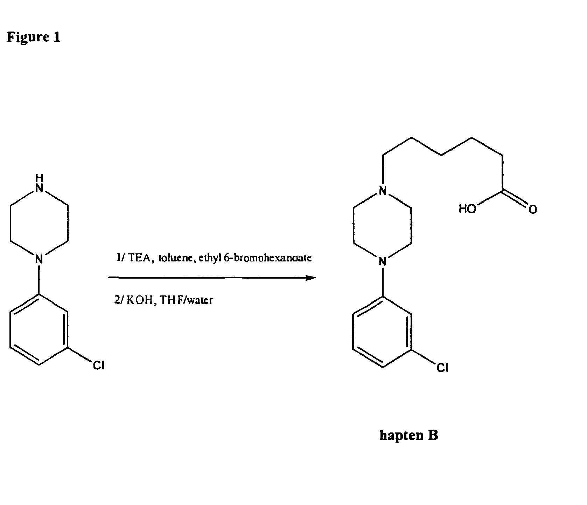

**Figure 1** Synthesis of the hapten 6-[N'-(3-chlorophenyl)-N-piperazinyl]hexanoic acid

### DETAILED DESCRIPTION OF THE INVENTION

A first aspect of the invention is the use of an antibody that binds to an epitope of N-(3-chlorophenyl)piperazine and/or an epitope of the N-(3-chlorophenyl)piperazine structure in an N'-substituted- N-(3-chlorophenyl)piperazine for use in detecting or determining N-(3-chlorophenyl)piperazine and/or N'-substituted- N-(3-chlorophenyl)piperazines in a solution or an *in vitro* sample taken from a patient. The antibody is raised against an immunogen of the structure 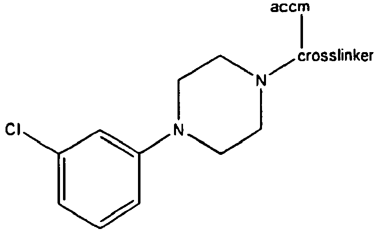 where the crosslinker joins the N'-atom of the piperazinyl ring to the accm, an antigenicity-conferring carrier material. The crosslinker of the immunogen from which the antibody is raised is -X-Y- where X is a C₁-C₁₀, more preferably a -C₂-C₆, most preferably a C₅, substituted or unsubstituted straight chain alkylene moiety, or arylene moiety, and Y (before conjugation with the accm) is selected from a carboxy, a dithiopyridyl, a maleimide, an amino, a hydroxyl, a thiol, a thioester or an aldehyde moiety, most preferably a carboxy moiety. The accm can be any material that makes all or part of the N-(3-chlorophenyl)piperazine structure susceptible to antibody recognition and binding. For example the accm can be a protein, a protein fragment, a synthetic polypeptide or a semisynthetic polypeptide. The antibody can be a monoclonal antibody, but is preferably a polyclonal antibody. The N'-substituted- N-(3-chlorophenyl)piperazine is preferably one or more of trazodone, nefazodone and 1-{3-[4-(3-chlorophenyl)-1-piperazinyl]propyl}-4-(2-phenoxyethyl)- 1,2,4-triazol-3,5-dione.

A further aspect of the invention is a method of detecting or determining N-(3-chlorophenyl)piperazine and/or N'-substituted- N-(3-chlorophenyl)piperazines in a solution or an individual, the method comprising contacting an *in vitro* sample taken from the individual or the solution with a conjugate, and an antibody that binds to an epitope of N-(3-chlorophenyl)piperazine and/or an epitope of the N-(3-chlorophenyl)piperazine structure in an N'-substituted- N-(3-chlorophenyl)piperazine, detecting the bound conjugate, and deducing from calibration values the presence of or amount of N-(3-chlorophenyl)piperazine and/or one or more N'-substituted- N-(3-chlorophenyl)piperazines. Preferably the N'-substituted- N-(3-chlorophenyl)piperazine is one or more of trazodone, nefazodone and 1-{3-[4-(3-chlorophenyl)-1-piperazinyl]propyl}-4-(2-phenoxyethyl)-1,2,4-triazol-3,5-dione.

Another aspect of the invention is a kit for detecting or determining N-(3-chlorophenyl)piperazine and/or N'-substituted- N-(3-chlorophenyl)piperazines, the kit including an antibody that binds to an epitope of N-(3-chlorophenyl)piperazine and/or an epitope of the N-(3-chlorophenyl)piperazine structure in an N'-substituted- N-(3-chlorophenyl)piperazine. Preferably the N'-substituted- N-(3-chlorophenyl)piperazine is one or more of trazodone, nefazodone and 1-{3-[4-(3-chlorophenyl)-1-piperazinyl]propyl}-4-(2-phenoxyethyl)- 1,2,4-triazol-3,5-dione. The kit may optionally include instructions for the use of said antibodies for detecting or determining N-(3-chlorophenyl)piperazine and/or N'-substituted- N-(3-chlorophenyl)piperazines. For the purposes of the invention, the solution may be, for example, a solution of cultured cells. A solution of the appropriate cultured cells would enable an *in vitro* assay to test for, for example, metabolic products and drug activity.

The sample can be any peripheral biological fluid but is preferably plasma or serum. The conjugates of the method are made up of haptens attached to labelling agents. The haptens of the conjugates are molecules that can bind to the antibodies of the method. The use of haptens, conjugates and antibodies in the context of immunoassays is well known in the art. Preferably, the labelling agent of the conjugates is selected from an enzyme, a luminescent substance, a radioactive substance, or a mixture thereof. More preferably, the labelling agent is an enzyme, preferably a peroxidase, most preferably horseradish peroxidase (HRP). Alternatively, or additionally, the luminescent substance may be a bioluminescent, chemiluminescent or fluorescent material.

### Methods and Results

### Preparation of Haptens, Immunogens and Conjugates

Although haptens provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and glycoproteins. Illustrative examples of useful carrier materials are bovine serum albumin (BSA), egg ovalbumin, bovine gamma globulin, bovine thyroglobulin (BTG), keyhole limpet haemocyanin (KLH) etc. Alternatively, synthetic poly(amino acids) having a sufficient number of available amino groups, such as lysine, may be employed, as may other synthetic or natural polymeric materials bearing reactive functional groups. In particular, carbohydrates, yeasts or polysaccharides may be conjugated to the hapten to produce an immunogen. The haptens can also be coupled to a detectable labelling agent such as an enzyme (for example, horseradish peroxidase), a substance having fluorescent properties or a radioactive label for the preparation of conjugates (or detection reagents) for use in the immunoassays. The fluorescent substance may be, for example, a monovalent residue of fluorescein or a derivative thereof. Immunogen formation involves conventional conjugation chemistry in which the oxygen of the hydroxyl group of Hapten A combines first with DCC and then NHS to form an ester with a powerful leaving group. Nucleophilic attack on the carbonyl of the ester functionality by a amine group on the protein (BSA or BTG), results in an amide bond and formation of the target immunogen. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS).

### General Procedure for MALDI-TOF Analysis of Immunogens.

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1µl) were analysed using a matrix of Sinapinic acid and bovine serum albumin (Fluka) was used as an external calibrant.

### Preparation of Antisera

In order to generate polyclonal antisera, the immunogen of the present invention is mixed with Freund's Adjuvant and the mixture is injected into a host animal, such as rabbit, sheep, mouse, guinea pig or horse (e.g. Seguela et a1.1984 for general immunisation protocol) Further injections (boosts) are made and serum is sampled for evaluation of the antibody titre. When the optimal titre has been attained, the host animal is bled to yield a suitable volume of specific antiserum. The degree of antibody purification required depends on the intended application. For many purposes, there is no requirement for purification, however, in other cases, such as where the antibody is to be immobilised on a solid support, purification steps can be taken to remove undesired material and eliminate nonspecific binding.

### Immunoassay Development

The process of developing an immunoassay is well known to the person skilled in the art. Briefly, for a competitive immunoassay in which the target analyte is a non-immunogenic molecule commonly referred to as a hapten, the following process is conducted: antibodies are produced by immunising an animal, preferably a mammalian animal, by repeated administration of an immunogen. The serum from the immunised animal is collected when the antibody titre is sufficiently high. A conjugate is added to a sample containing the target analyte and the raised antibodies, and the conjugate and analyte compete for binding to the antibodies. The process may comprise fixing said serum antibodies to a backing substrate such as a polystyrene solid support or a biochip. The antibodies can be polyclonal or monoclonal. The signal emitted in the immunoassay is proportionate to the amount of conjugate bound to the antibodies which in turn is inversely proportionate to the analyte concentration. The signal can be detected or quantified by comparison with a calibrator.

### Example 1: Preparation of 6-[N'-(3-chlorophenyl)-N-piperazinyl]hexanoic acid (hapten B)

To a solution of ethyl 6-[N'-(3-chlorophenyl)-N-piperazinyl]hexanoate (10g, 0.029mol) in a mixture of THF / water (1 /1) was added potassium hydroxide (12g, 0.087mol) and the mixture was stirred at room temperature overnight. The THF was removed under vacuum and the solution was acidified with HCl (2N). The precipitate was filtered, washed by water and dried. Recrystallization with methanol gave 6-[N'-(3-chlorophenyl)-N-piperazinyl]hexanoic acid (hapten B) (6.5g). ¹³C NMR (δ: ppm): 177.61, 152.78, 136.6, 132.15, 122.18, 118.15, 116.29, 58.21, 53.3, 47.86, 34.83, 27.43, 25.77, 25.18.

### Example 2: Conjugation of 6-[N'-(3-chlorophenyl)-N-piperazinyl]hexanoic acid to BSA

To a cooled solution at 0°C of hapten B (37.28mg, 0.12mmol) in DMF (3ml) under nitrogen was added tri-n-butylamine (31.42µl, 0.132mmol) and isobutyl chloroformate (IBCF) (17.02µl, 0.132mmol). The mixture was stirred at 0°C for 15 minutes and then added drop-wise to a cooled solution of BSA (100mg) in sodium bicarbonate (100mM, 10ml) and the mixture was stirred at 4°C overnight. The solution was then dialysed against 50mM phosphate buffer pH 7.2 (3 changes) for 24 hours at 4°C, and freeze-dried. MALDI results showed 22.02 molecules of hapten B had been conjugated to one molecule of BSA.

### Example 3: Conjugation of 6-[N'-(3-chlorophenyl)-N-piperazinyl]hexanoic acid to BTG

To a cooled solution at 0°C of hapten B (65.09mg, 0.203mmol) in DMF (3ml) under nitrogen was added tri-n-butylamine (53.1µl, 0.223mmol) and isobutyl chloroformate (IBCF) (28.78µl, 0.223mmol). The mixture was stirred at 0°C for 15 minutes and then added drop-wise to a cooled solution of BTG (150mg) in sodium bicarbonate (100mM, 10ml) and the mixture was stirred at 4°C overnight. The solution was then dialysed with 50mM phosphate buffer pH 7.2 (3 changes) for 24 hours at 4°C, and freeze-dried.

### Example 4: Conjugation of 6-[N'-(3-chlorophenyl)-N-piperazinyl]hexanoic acid to HRP

To a cooled solution at 0°C of hapten B (2mg) in DMF (200µl) under nitrogen was added tri-n-butylamine (38µl) and isobutyl chloroformate (IBCF) (2µl). The mixture was stirred at 0°C for 10 minutes and then added drop-wise to a cooled solution of HRP (200mg) in water (800 µl) and the reaction mixture was incubated in the dark at room temperature overnight. Excess hapten was removed with double PD-10 columns (Pharmacia) in series, pre-equilibrated with PBS at pH 7.2. The hapten-HRP conjugate was then dialysed overnight with 10L of PBS at pH 7.2 at 4°C.

### Example 5: Development of ELISAs for N-(3-chlorophenyl)piperazine and N'-substituted-N-(3-chlorophenyl)piperazines

Trazodone and N-(3-chlorophenyl)piperazine were coupled by way of a crosslinker to bovine thyroglobulin (BTG). The resulting immunogens were administered separately to adult sheep on a monthly basis to provide target-specific polyclonal antisera. IgG was extracted from the antisera via Caprylic acid/ammonium sulphate precipitation of immunoglobulin. Microtitre plates (Thermo Scientific, 95029180) were coated with antibody (125µl) in coating buffer (10mM Tris pH 8.5) at 37°C for 2 hours. Antibody was coated at 2.5µg/ml. The plates were then washed. 50µl of sample/standard (trazodone, Sigma T6154-1g; N-(3-chlorophenyl)piperazine, Alfa-Aesar L01772; nefazodone, Sigma N5536) was added to the appropriate wells in triplicate, followed by 75µl of hapten-HRP conjugate and incubated at 25°C for 1 hour. The plates were then washed and 125µl of TMB (Randox, 4380-15) was added to each well and left at room temperature for 20mins in the dark. The reaction was stopped using 125µl of 0.2M sulphuric acid. The absorbancies were read at 450nm with an ELISA microplate reader (BIO-TEK Instruments, EL340) and the means calculated. Antibody specificity and sensitivity were then determined

### Results

Competitive ELISA results in Table 1 highlight the broad specificity of the antibody of the invention towards N-(3-chlorophenyl)piperazine and N'-substituted- N-(3-chlorophenyl)piperazines.

**Table 1**

| | | **Trazodone** | | **mCPP** | | **Nefazodone** | |
|---|---|---|---|---|---|---|---|
| | **Standard concⁿ ng/ml** | **A₄₅₀** | **%B/B₀** | **A₄₅₀** | **% B/B₀** | **A₄₅₀** | **%B/B₀** |
| | 0.000 | 2.184 | 100 | 2.133 | 100 | 2.023 | 100 |
| | 0.156 | 1.456 | 67 | 1.821 | 85 | 1.850 | 91 |
| | 0.313 | 1.271 | 58 | 1.679 | 79 | 1.671 | 83 |
| | 0.625 | 1.041 | 48 | 1.534 | 72 | 1.467 | 73 |
| | 1.250 | 0.763 | 35 | 1.380 | 65 | 1.168 | 58 |
| | 2.500 | 0.551 | 25 | 1.183 | 55 | 0.923 | 46 |
| | 5.000 | 0.398 | 18 | 1.027 | 48 | 0.683 | 34 |
| | 10.000 | 0.287 | 13 | 0.888 | 42 | 0.494 | 24 |
| **IC₅₀** | | 0.532 | | 8.476 | | 3.901 | |
| **%CR** | | 733 | | 46 | | 100 | |

| | | | | | | | |
|---|---|---|---|---|---|---|---|
| A₄₅₀ =absorbance at 450 nm; B = absorbance at 450 nm at x ng/ml standard concentration B₀ = absorbance at 450 nm at 0 ng/ml standard concentration; IC₅₀ = standard concentration which produces 50% B/B₀; %CR = percentage cross-reactivity based on 100% specificity to nefazodone. | | | | | | | |

## Claims

1. An antibody for use in detecting or determining N-(3-chlorophenyl)piperazine and N'-substituted-N-(3-chlorophenyl)piperazines in a solution or an *in vitro* sample taken from a patient **characterized by** being raised from an immunogen of structure I and further **characterized by** being able to bind to an epitope of N-(3-chlorophenyl)piperazine or the N-(3-chlorophenyl)piperazine moiety of an N'-substituted N-(3-chlorophenyl)piperazine. 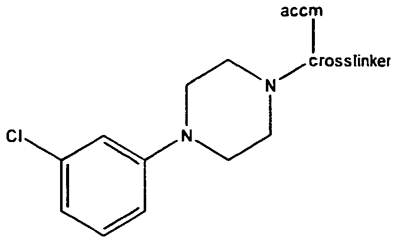 Structure I, wherein the "accm" abbreviation stands for antigenicity conferring carrier material.

2. The antibody of Claim 1 in which the crosslinker of the immunogen from which the antibody is raised is -X-Y- where X is a C₁-C₁₀, more preferably a -C₂-C₆, most preferably a C₅, substituted or unsubstituted straight chain alkylene moiety, or arylene moiety, and Y (before conjugation with the accm) is selected from a carboxy, a dithiopyridyl, a maleimide, an amino, a hydroxyl, a thiol, a thioester or an aldehyde moiety, most preferably a carboxy moiety.

3. The antibody of Claims 1 or 2 in which the N'-substituted -N-(3-chlorophenyl)piperazine is one or more of trazodone, nefazodone and 1-{3-[4-(3-chlorophenyl)-1-piperazinyl]propyl}-4-(2-phenoxyethyl)-1,2,4-triazol-3,5-dione.

4. A method of detecting or determining N-(3-chlorophenyl)piperazine and N'-substituted- N-(3-chlorophenyl)piperazines in an individual or a solution, the method comprising contacting an *in vitro* sample taken from the individual or a solution with a conjugate, and the antibody of Claims 1 or 2, detecting the bound conjugate, and deducing from calibration values the presence of or amount of N-(3-chlorophenyl)piperazine and/or one or more N'-substituted- N-(3-chlorophenyl)piperazines.

5. The method of Claim 4 in which the N'-substituted- N-(3-chlorophenyl)piperazine is one or more of trazodone, nefazodone and 1-{3-[4-(3-chlorophenyl)-1-piperazinyl]propyl}-4-(2-phenoxyethyl)- 1,2,4-triazol-3,5-dione.

6. A kit for detecting or determining N-(3-chlorophenyl)piperazine and N'-substituted- N-(3-chlorophenyl)piperazines, the kit including the antibody of Claims 1 or 2.

7. The kit of Claim 6 in which the N'-substituted- N-(3-chlorophenyl)piperazine is one or more of trazodone, nefazodone and 1-{3-[4-(3-chlorophenyl)-1-piperazinyl]propyl}-4-(2-phenoxyethyl)- 1,2,4-triazol-3,5-dione.

## Patentansprüche

1. Antikörper zur Verwendung beim Nachweisen oder Bestimmen von N-(3-Chlorphenyl)piperazin und N'-substituierten-N-(3-Chlorphenyl)piperazinen in einer Lösung oder einer *In-vitro*-Probe von einem Patienten, **dadurch gekennzeichnet, dass** er von einem Immunogen der Struktur I erzeugt wird, und ferner **dadurch gekennzeichnet, dass** er sich an ein Epitop von N-(3-Chlorphenyl)piperazin oder den N-(3-Chlorphenyl)piperazin-Anteil eines N'-substituierten N-(3-Chlorphenyl)piperazins binden kann. 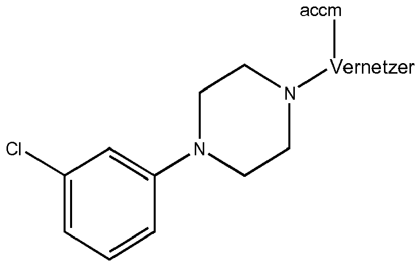 Struktur I, wobei die Abkürzung "accm" für Antigenität übertragendes Trägermaterial steht.

2. Antikörper nach Anspruch 1, wobei der Vernetzer des Immunogens, von dem der Antikörper erzeugt wird, -X-Y- ist, wobei X ein substituierter oder unsubstituierter, geradkettiger C₁-C₁₀, bevorzugter -C₂-C₆, am bevorzugtesten C₅ Alkylenanteil oder Arylenanteil ist und Y (vor der Konjugation mit accm) aus einem Carboxy-, Dithiopyridyl-, Maleimid-, Amino-, Hydroxyl-, Thiol-, Thioester- oder Aldehydanteil, am bevorzugtesten aus einem Carboxyanteil, ausgewählt ist.

3. Antikörper nach Anspruch 1 oder 2, wobei das N'-substituierte -N-(3-Chlorphenyl)piperazin eines oder mehrere aus Trazodon, Nefazodon und 1-{3-[4-(3-Chlorphenyl)-1-piperazinyl]propyl}-4-(2-phenoxyethyl)- 1,2,4-triazol-3,5-dion ist.

4. Verfahren zum Nachweisen oder Bestimmen von N-(3-Chlorphenyl)piperazin und N'-substitutierten- N-(3-Chlorphenyl)piperazinen in einem Individuum oder einer Lösung, wobei das Verfahren das Inkontaktbringen einer In-vitro-Probe von dem Individuum oder einer Lösung mit einem Konjugat und dem Antikörper aus Anspruch 1 oder 2, Detektieren des gebundenen Konjugats und Herleiten der Anwesenheit oder einer Menge von N-(3-Chlorphenyl)piperazin und/oder von einem oder mehreren N'-substituierten- N-(3-Chlorphenyl)piperazinen anhand Kalibrierungswerten beinhaltet.

5. Verfahren nach Anspruch 4, wobei das N'-substituierte- N-(3-Chlorphenyl)piperazin eines oder mehrere aus Trazodon, Nefazodon und 1-{3-[4-(3-Chlorphenyl)-1-piperazinyl]propyl}-4-(2-phenoxyethyl)- 1,2,4-triazol-3,5-dion ist.

6. Kit zum Nachweisen oder Bestimmen von N-(3-Chlorphenyl)piperazin und N'-substituierten- N-(3-Chlorphenyl)piperazinen, wobei der Kit den Antikörper aus Anspruch 1 oder 2 beinhaltet.

7. Kit nach Anspruch 6, in dem das N'-substituierte- N-(3-Chlorphenyl)piperazin eines oder mehrere aus Trazodon, Nefazodon und 1-{3-[4-(3-Chlorphenyl)-1-piperazinyl]propyl}-4-(2-phenoxyethyl)- 1,2,4-triazol-3,5-dion ist.

## Revendications

1. Anticorps pour une utilisation dans la détection ou la détermination de N-(3-chlorophényl)pipérazine et de N-(3-chlorophényl)pipérazines N'-substituées dans une solution ou dans un échantillon *in vitro* prélevé sur un patient **caractérisé en ce qu'**il provient d'un immunogène de structure I est **caractérisé en outre en ce qu'**il est capable de se lier à un épitope de N-(3-chlorophényl)pipérazine ou au fragment N-(3-chlorophényl)pipérazine d'une N-(3-chlorophényl)pipérazine N'-substituée. 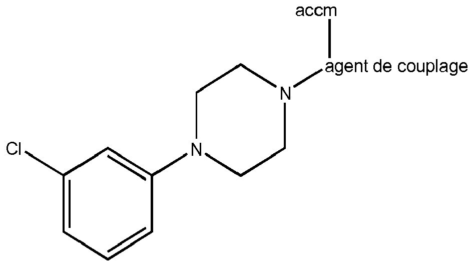 Structure I, où l'abréviation « accm » désigne un matériau porteur conférant l'antigénicité.

2. Anticorps selon la revendication 1, dans lequel l'agent de couplage de l'immunogène d'où provient l'anticorps est -X-Y-, X étant un fragment alkylène ou un fragment arylène à chaîne linéaire substitué ou non substitué en C₁ à C₁₀, plus préférablement en C₂ à C₆, le plus préférablement en C₅, et Y (avant d'être conjugué avec l'accm) est choisi parmi un fragment carboxy, dithiopyridyle, maléimide, amino, hydroxyle, thiol, thioester ou aldéhyde, le plus préférablement un fragment carboxy.

3. Anticorps selon la revendication 1 ou 2, dans lequel la N-(3-chlorophényl)pipérazine N-substituée est une ou plusieurs de la tradozone, de la néfazodone et de la 1-{3-[4-(3-chlorophényl)-1-pipérazinyl]propyl}-4-(2-phénoxyéthyl)-1,2,4-triazol-3,5-dione.

4. Procédé de détection ou de détermination de N-(3-chlorophényl)pipérazine et de N-(3-chlorophényl)pipérazines N'-substituées chez un individu ou dans une solution, le procédé comprenant la mise en contact d'un échantillon *in vitro* prélevé sur un patient ou dans une solution avec un conjugué, et l'anticorps selon la revendication 1 ou 2, la détection du conjugué lié et la déduction d'après les valeurs d'étalonnage de la présence ou de la quantité de N-(3-chlorophényl)pipérazine et/ou d'une ou plusieurs N-(3-chlorophényl)pipérazines N'-substituées.

5. Procédé selon la revendication 4, dans lequel la N-(3-chlorophényl)pipérazine N-substituée est une ou plusieurs de la tradozone, de la néfazodone et de la 1-{3-[4-(3-chlorophényl)-1-pipérazinyl]propyl}-4-(2-phénoxyéthyl)-1,2,4-triazol-3,5-dione.

6. Kit pour détecter ou déterminer la N-(3-chlorophényl)pipérazine et les N-(3-chlorophényl)pipérazines N'-substituées, le kit comprenant l'anticorps selon la revendication 1 ou 2.

7. Kit selon la revendication 6, dans lequel la N-(3-chlorophényl)pipérazine N'-substituée est une ou plusieurs de la tradozone, de la néfazodone et de la 1-{3-[4-(3-chlorophényl)-1-pipérazinyl]propyl}-4-(2-phénoxyéthyl)-1,2,4-triazol-3,5-dione.
